# EUROPEAN PATENT SPECIFICATION

(11) **EP 0 934 259 B1**
(45) Date of publication and mention of the grant of the patent: **18.09.2002**
(21) Application number: 97912674.5
(22) Date of filing: 08.10.1997
(51) Int. Cl.: C07C 311/20, A61K 31/215

(54) **BETA-SULFONAMIDO HYDROXAMIC ACIDS AS MATRIX METALLOPROTEINASE AND TACE INHIBITORS**
BETA-SULFONAMIDO HYDROXAMSÄURE ALS MATRIX METALLOPROTEINASE UND ALS TACE INHIBITOREN
ACIDES BETA-SULFONAMIDO HYDROXAMIQUES UTILISES COMME INHIBITEURS DE METALLOPROTEASES MATRICIELLES ET DE TACE

(30) Priority: 16.10.1996 US 729359
(43) Date of publication of application: 11.08.1999
(73) Proprietor: American Cyanamid Company, Madison, New Jersey 07940-0874 (US)
(72) Inventor: LEVIN, Jeremy, Ian, Nanuet, NY 10954 (US); ZASK, Arie, New York, NY 10128 (US); GU, Yansong, Pearl River, NY 10965 (US)
(74) Representative: Talbott, Dawn Jacqueline
(86) International application number: US9718170
(87) International publication number: WO98016506

(56) References cited:
- EP-A- 0 606 046
- WO-A-94/22309
- WO-A-96/29313

## Description

### Background of the Invention

The present invention relates to the discovery of novel, low molecular weight, non-peptide inhibitors of matrix metalloproteinases (e.g. gelatinases, stromelysins and collagenases) and TNF-α converting enzyme (TACE, tumor necrosis factor-α converting enzyme) which are useful for the treatment of diseases in which these enzymes are implicated such as arthritis, tumor metastasis, tissue ulceration, abnormal wound healing, periodontal disease, bone disease, proteinuria, aneurysmal aortic disease, degenerative cartilage loss following traumatic joint injury, demyelinating diseases of the nervous system and HIV infection.

Matrix metalloproteinases (MMPs) are a group of enzymes that have been implicated in the pathological destruction of connective tissue and basement membranes [Woessner, J.F., Jr. *FASEB J*. **1991,** *5*, 2145;.Birkedal-Hansen, H.; Moore, W.G.I.; Bodden, M.K.; Windsor, L.J.; Birkedal-Hansen, B.; DeCarlo, A.; Engler, J.A. *Crit. Rev. Oral Biol. Med*. **1993**, *4*, 197; Cawston, T.E. *Pharmacol*. *Ther*. **1996**, *70*, 163; Powell, W.C.; Matrisian, L.M. *Cur. Top. Microbial. and Immunol*. **1996**, *213*, 1]. These zinc containing endopeptidases consist of several subsets of enzymes including collagenases, stromelysins and gelatinases. Of these classes, the gelatinases have been shown to be the MMPs most intimately involved with the growth and spread of tumors, while the collagenases have been associated with the pathogenesis of osteoarthritis [Howell, D.S.; Pelletier, J.-P. In *Arthritis and Allied Conditions*; McCarthy, D.J.; Koopman, W.J., Eds.; Lea and Febiger; Philadelphia, **1993;** *12th Edition* Vol. 2, pp. 1723; Dean, D.D. *Sem. Arthritis Rheum.* **1991,** *20*, 2; Crawford, H.C; Matrisian, L.M. *Invasion* Metast. ***1994***-**95,** *14,* 234; Ray, J.M.; Stetler-Stevenson, W.G. *Exp*. *Opin*. *Invest. Drugs,* **1996,** *5*, 323].

It is known that the level of expression of gelatinase is elevated in malignancies, and that gelatinase can degrade the basement membrane which may lead to tumor metastasis [Powell, W.C.; Matrisian, L.M. *Cur. Top. Microbiol. and Immunol.* **1996,** *213, 1;* Crawford, H.C; Matrisian, L.M. *Invasion Metast.* **1994-95**, *14,* 234; Ray, J.M.; Stetler-Stevenson, W.G. *Exp. Opin. Invest. Drugs*, **1996**, *5,* 323; Himelstein, B.P.; Canete-Soler, R.; Bernhard, E.J.; Dilks, D.W.; Muschel, R.J. *Invasion Metast.* **1994-95,** *14*, 246; Nuovo, G.J.; MacConnell, P.B.; Simsir, A.; Valea, F.; French, D.L. *Cancer Res.* **1995,** *55*, 267-275; Walther, M.M.; Levy, A.; Hurley, K.; Venzon, D.; Linehen, W.M.; Stetler-Stevenson, W. *J. Urol.* **1995,** *153 (Suppl. 4*), 403A; Tokuraku, M; Sato, H.; Murakami, S.; Okada, Y.; Watanabe, Y.; Seiki, *M. Int. J. Cancer,* **1995,** *64, 355;* Himelstein, B.; Hua, J.; Bernhard, E.; Muschel, RJ. *Proc. Am. Assoc. Cancer Res. Ann. Meet.* **1996**, *37,* 632; Ueda, Y.; Imai, K.; Tsuchiya, H.; Fujimoto, N.; Nakanishi, I.; Katsuda, S.; Seiki, M.; Okada, Y. *Am. J. Pathol.* **1996**, *148,* 611; Gress, T.M.; Mueller-Pillasch, F.; Lerch, M.M.; Friess, H.; Buechler, M.; Adler, G. *Int. J. Cancer,* **1995,** *62,* 407; Kawashima, A.; Nakanishi, I.; Tsuchiya, H.; Roessner, A.; Obata, K.; Okada, Y. *Virchows Arch*., **1994,** *424,* 547-552.]. Angiogenesis, required for the growth of solid tumors, has also recently been shown to have a gelatinase component to its pathology [Crawford, H.C; Matrisian, L.M. *Invasion Metast*. **1994-95**, *14*, 234; Ray, J.M.; Stetler-Stevenson, W.G. *Exp. Opin. Invest. Drugs,* **1996,** *5*, 323.]. Furthermore, there is evidence to suggest that gelatinase is involved in plaque rupture associated with atherosclerosis [Dollery, C.M.; McEwan, J.R.; Henney, A.M. *Circ. Res.* **1995,** *77,* 863; Zempo, N.; Koyama, N.; Kenagy, R.D.; Lea, H.J.; Clowes, A.W. *Arterioscler. Thromb. Vasc. Biol.* **1996,** *16*, 28; Lee, R.T.; Schoen, F.J.; Loree, H.M.; Lark, M.W., Libby, P. *Arterioscler. Thromb. Vasc. Biol.* **1996,** *16*, 1070.]. Other conditions mediated by MMPs are restenosis, MMP-mediated osteopenias, inflammatory diseases of the central nervous system, skin aging, tumor growth, osteoarthritis, rheumatoid arthritis, septic arthritis, comeal ulceration, abnormal wound healing, bone disease, proteinuria, aneurysmal aortic disease, degenerative cartilage loss following traumatic joint injury, demyelinating diseases of the nervous system, cirrhosis of the liver, glomerular disease of the kidney, premature rupture of fetal membranes, inflammatory bowel disease, periodontal disease, age related macular degeneration, diabetic retinopathy, proliferative vitreoretinopathy, retinopathy of prematurity, ocular inflammation, keratoconus, Sjogren's syndrome, myopia, ocular tumors, ocular angiogenesis/neovascularization and corneal graft rejection.

The hypothesis that MMPs are important mediators of the tissue destruction that occurs in arthritis has long been considered, since it was first recognized that these enzymes are capable of degrading collagens and proteoglycans which are the major structural components of cartilage [Sapolsky, A.I.; Keiser, H.; Howell, D.S.; Woessner, J.F., Jr.; *J. Clin*. *Invest.* **1976,** *58*, 1030; Pelletier, J.-P.; Martel-Pelletier, J.; Howell, D.S.; Ghandur-Mnaymneh, L; Enis, J.E.; Woessner, J.F., Jr., *Arthritis Rheum*. **1983,** 26, 63.], and continues to develop as new MMPs are identified. For example, collagenase-3 (MMP-13) was cloned from breast cancer cells in 1994, and the first report that it could be involved in arthritis appeared in 1995 [Freiji, J.M.; Diez-Itza, I.; Balbin, M.; Sanchez, L.M.; Blasco, R.; Tolivia, J.; Lopez-Otin, C. *J. Biol*. *Chem.* **1994,** *269,* 16766; Flannery, C.R.; Sandy, J.D. **102-17,** *41st Ann. Meet. Orth*. *Res*. *Soc.* Orlando, FL. February 13-16, 1995.]. Evidence is accumulating that implicates MMP-13 in the pathogenesis of arthritis. A major structural component of articular cartilage, type II collagen, is the preferred substrate for MMP-13 and this enzyme is significantly more efficient at cleaving type II collagen than the other collagenases [Knauper, V.; Lopez-Otin, C.; Smith, B.; Knight, G.; Murphy, G. *J. Biol*. *Chem*., **1996,** *271,* 1544-1550; Mitchell, P.G.; Magna, H.A.; Reeves, L.M.; Lopresti-Morrow, L.L.; Yocum, S.A.; Rosner, P.J.; Geoghegan, K.F.; Hambor, J.E. *J*. *Clin*. *Invest.* **1996,** *97,* 761.]. MMP-13 is produced by chondrocytes, and elevated levels of MMP-13 has been found in human osteoarthritic tissues [Reboul, P.; Pelletier, J-P.; Hambor, J.; Magna, H.; Tardif, G.; Cloutier, J-M.; Martel-Pelletier, *J. Arthritis Rheum.* **1995,** *38 (Suppl. 9),* S268;Shlopov, B.V.; Mainardi, C.L.; Hasty, K.A. *Arthritis Rheum.* **1995,** *38 (Suppl.* 9), S313; Reboul, P.; Pellener. J-P.; Tardif, G.; Cloutier, J-M.; Martel-Pelletier, J. *J. Clin. Invest.* **1996,** *97,* 2011]. Potent inhibitors of MMPs were described over 10 years ago, but the poor bioavailability of these early peptidic, substrate mimetic MMP inhibitors precluded their evaluation in animal models of arthritis. More bioavailable, non-peptidic MMP inhibitors may be preferred for the treatment of diseases mediated by MMPs.

TNF-α converting enzyme catalyzes the formation of TNF-α from membrane bound TNF-α precursor protein. TNF-α is a pro-inflammatory cytokine that is now thought to have a role in rheumatoid arthritis, septic shock, graft rejection, insulin resistance and HIV infection in addition to its well documented antitumor properties. For example, research with anti-TNF-α antibodies and transgenic animals has demonstrated that blocking the formation of TNF-α inhibits the progression of arthritis [Rankin, E.C.; Choy, E.H.; Kassimos, D.; Kingsley, G.H.; Sopwith, A.M.; Isenberg, D.A.; Panayi, G.S. *Br. J. Rheumatol*. **1995,** *34,* 334; *Pharmaprojects*, **1996,** Therapeutic Updates 17 *(Oct.),* au197-M2Z.]. This observation has recently been extended to humans as well. Other conditions mediated by TNF-α are congestive heart failure, cachexia, anorexia, inflammation, fever, inflammatory disease of the central nervous system, and inflammatory bowel disease.

It is expected that small molecule inhibitors of gelatinase and TACE therefore have the potential for treating a variety of disease states. While a variety of MMP and TACE inhibitors have been identified and disclosed in the literature, the vast majority of these molecules are peptidic or peptide-like compounds that may have bioavailability and pharmcokinetic problems that would limit their clinical effectiveness. Low molecular weight, potent, long-acting, orally bioavailable inhibitors of gelatinases, collagenases and/or TACE are therefore highly desirable for the potential chronic treatment of the above mentioned disease states. Several non-peptidc, sulfur-containing hydroxamic acids have recently been disclosed and are listed below.

U. S. patents 5,455,258, 5,506,242 and 5,552,419, as well as European patent application EP606,046A1 and WIPO international publications WO96/00214 and WO97/22587 disclose non-peptide matrix metalloproteinase inhibitors of which the compound CGS27023A is representative. The discovery of this type of MMP inhibitor is further detailed by MacPherson, *et*. *al*. in *J. Med*. *Chem.,* (1997), 40, 2525. Additional publications disclosing sulfonamide based MMP inhibitors which are variants of the sulfonamide-hydroxamate shown below, or the analogous sulfonamide-carboxylates, are European patent application EP-757984-A1 and WIPO international publications WO95/35275, WO95/35276, WO96/27583, WO97/19068 and WO97/27174.

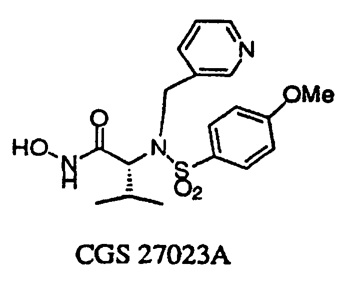

Publications disclosing β-sulfonamide-hydroxamate MMP inhibitor analogs of CGS 27023A in which the carbon alpha to the hydroxamic acid has been joined in a ring to the sulfonamide nitrogen, as shown below, include WIPO international publications WO96/33172 and WO97/20824.

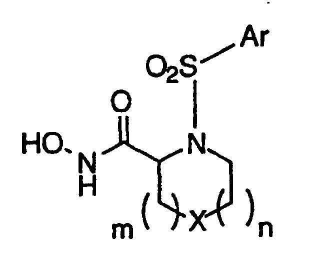

The German patent application DE19,542,189-A1 discloses additional examples of cylic sulfonamides as MMP inhibitors. In this case the sulfonamide-containing ring is fused to a phenyl ring to form an isoquinoline.

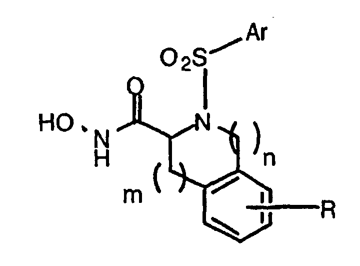

Analogs of the sulfonamide-hydroxamate MMP inhibitors in which the sulfonamide nitrogen has been replaced by a carbon atom, as shown in the general structure below, are European patent application EP-780386-A1 and WIPO international publication WO97/24117.

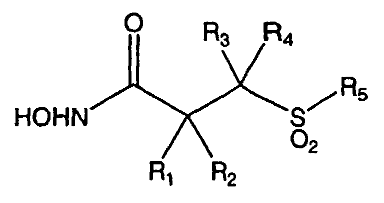

### Summary of the Invention

The TACE and MMP inhibiting β-sulfonamido hydroxamic acids of the present invention are represented by the formula 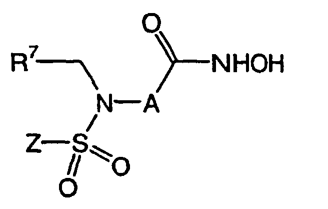 where the hydroxamic acid moiety and the sulfonamido moiety are bonded to adjacent carbons of group A where:
A is a 5 to 7 membered, monocyclic, non-aromatic, heterocyclic ring having from 1 to 2 heteroatoms independently selected from N, O, and S, optionally substituted by R¹, R², R³ and R⁴;
or a -C₃-C₇-cycloalkyl containing 0-2 double bonds and optionally substituted with R¹, R², R³ and R⁴;
or -CHR⁵=CHR⁶-;
Z is aryl, heteroaryl, or heteroaryl fused to a phenyl,
   where aryl is phenyl or naphthyl optionally substituted by R¹, R², R³ and R⁴;
   heteroaryl is a 5-6 membered heteroaromatic ring having from 1 to 3 heteroatoms independently selected from N, O, and S, and optionally substituted by R¹, R², R³ and R⁴;
   and when heteroaryl is fused to phenyl, either or both of the rings can be optionally substituted by R¹, R², R³ and R⁴;
R¹, R², R³ and R⁴ are independently -H, -COR⁵, -F,-Br, -Cl, -I, -C(O)NR⁵OR⁶, -CN, -OR⁵, -C₁-C₄-perfluoroalkyl, -S(O)ₓR⁵ where x is 0-2, -OPO(OR⁵)OR⁶, -PO(OR⁶)R⁵, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, -NR⁵C(=NR⁶)NR⁵R⁶; 3-6 membered cycloheteroalkyl having one to three heteroatoms independently selected from N, O, and S and optionally having 1 or 2 double bonds and optionally substituted by one to three groups each selected independently from R⁵;
-aryl or heteroaryl as defined above, -SO₂NHCOR⁵ or -CONHSO₂R⁵ where R⁵ is not hydrogen; -tetrazol-5-yl, -SO₂NHCN, -SO₂NHCONR⁵R⁶ or straight chain or branched -C₁-C₆ alkyl, -C₂-C₆-alkenyl, -C₂-C₆-alkynyl, or -C₃-C₆-cycloalkyl optionally having 1 or 2 double bonds each optionally substituted with
   -COR⁵, -CN, -C₂-C₆alkenyl, -C₂-C₆alkynyl, -OR⁵, -C₁-C₄-perfluoroalkyl, -S(O)ₓR⁵ where x is 0-2, -OC(O)NR⁵R⁶, -COOR⁵, -CONR⁵R⁶, -SO₃H, -NR⁵R⁶,-NR⁵COR⁶, -NR⁵COOR⁶,
   -SO₂NR⁵R⁶, -NO₂, -N(R⁵)SO₂R⁶, -NR⁵CONR⁵R⁶, -C₃-C₆ cycloalkyl as defined above,
   3-6 membered cycloheteroalkyl as defined above, aryl or heteroaryl as defined above, -SO₂NHCOR⁵ or -CONHSO₂R⁵ where R⁵ is not hydrogen; -PO(OR⁵)OR⁶, -PO(OR⁶)R⁵, -tetrazol-5-yl, -C(O)NR⁵OR⁶, NR⁵C(=NR⁶)NR⁵R⁶, -SO₂NHCONR⁵R⁶ or -SO₂NHCN;
with the proviso that when R¹ and R² are on adjacent carbons of A, R¹ and R² together with the carbons to which they are attached can form a 5-7 membered saturated or unsaturated non-aromatic monocyclic heterocyclic ring, each having from 1 to 2 heteroatoms independently selected from N, O, and S, wherein said heterocyclic or heteroaryl ring may be optionally substituted by one to four groups each selected independently from R⁴; or R¹ and R² together with the carbons to which they are attached can form a 5-7 membered saturated or unsaturated carbocyclic ring or an aryl ring wherein said carbocyclic or aryl ring may be optionally substituted by one to four groups each selected independently from R⁴;
R⁵ and R⁶ are independently defined as H, aryl and heteroaryl as defined above, -C₃-C₆-cycloalkyl as defined above, -C₃-C₆-cycloheteroalkyl as defined above, -C₁-C₄-perfluoroalkyl, or straight chain or branched -C₁-C₆ alkyl, -C₂-C₆-alkenyl, or -C₂-C₆-alkynyl each optionally substituted with -OH,
   -COR⁸, -CN, -C(O)NR⁸OR⁹, -C₂-C₆-alkenyl, -C₂-C₆-alkynyl, -OR⁸, -C₁-C₄-perfluoroalkyl, -S(O)ₓR⁸ where x is 0-2, -OPO(OR⁸)OR⁹, -PO(OR⁸)R⁹, -OC(O)NR⁸R⁹, -COOR⁸, - -CONR⁸R⁹, -SO₃H, -NR⁸R⁹,-NCOR⁸R⁹, -NR⁸COOR⁹, -SO₂NR⁸R⁹, -NO₂, -N(R⁸)SO₂R⁹, -NR⁸CONR⁸R⁹, -C₃-C₆ cycloalkyl as defined above, -C₃-C₆- cycloheteroalkyl as defined above, -aryl or heteroaryl as defined above, -SO₂NHCOR⁸ or -CONHSO₂R⁸ where R⁸ is not hyrdogen, -tetrazol-5-yl, -NR⁸C(=NR⁹)NR⁸R⁹, -SO₂NH_{C}ONR⁸R⁹, -SO₂NHCN;
R⁷ is hydrogen, straight chain or branched -C₁-C₆-alkyl, -C₂-C₆-alkenyl, or -C2-C₆-alkynyl each optionally substituted with -OH, -COR⁵, -CN, -C2-C6-alkenyl, -C₂-C₆-alkynyl, -OR⁵, -C₁-C₄-perfluoroalkyl, -S(O)ₓR⁵ where x is 0-2, -OPO(OR⁵)OR⁶, -PO(OR⁵)R⁶, -OC(O)NR⁵R⁶, -COOR⁵, -CONR⁵R⁶, -SO₃H, -NR⁵R⁶,-NR⁵COR⁶, -NR⁵COOR⁶, SO₂NR⁵R⁶, -NO₂, -N(R⁵)SO₂R⁶, -NR⁵CONR⁵R⁶, -C₃-C₆ cycloalkyl as defined above, -C₃-C₆-cycloheteroalkyl as defined above, -aryl or heteroaryl as defined above, -SO₂NHCOR⁵ or -CONHSO₂R⁵ where R⁵ is not hydrogen, -tetrazol-5-yl, -NR⁵C(=NR6)NR⁵R⁶, -C(O)N R⁵OR⁶, -SO₂NHCONR⁵R⁶or -SO₂NHCN;
   or R⁷ is phenyl or naphthyl, optionally substituted by R¹, R², R³ and R⁴ or a 5 to 6 membered heteroaryl group having 1 to 3 heteroatoms selected independently from N, O, and S and optionally substituted by R¹, R², R³ and R⁴;
or R⁷ is C₃-C₆ cycloalkyl or 3-6 membered cycloheteroalkyl as defined above;
or R⁷-CH₂-N-A-, where A is as defined above, can form with a carbon atom of A adjacent to the carbon bearing the sulfonamido group, a non-aromatic fused, 7-10 membered heterocyclic ring, optionally containing an additional heteroatom selected from O, S and N wherein said heterocyclic ring may be optionally fused to a benzene ring;
R⁸ and R⁹ are independently H, aryl or heteroaryl as defined above, -C₃-C₇-cycloalkyl or cycloheteroalkyl as defined above, -C₁-C₄-perfluoroalkyl;
or straight chain or branched -C₁-C₆-alkyl, -C₂-C₆-alkenyl, or -C₂-C₆-alkynyl, each optionally substituted with hydroxy, alkoxy, aryloxy, -C₁-C₄-perfluoroalkyl, amino, mono- and di-C₁-C₆-alkylamino, carboxylic acid, carboalkoxy and carboaryloxy, nitro, cyano, carboxamido primary, mono- and di-C₁-C₆-alkylcarbamoyl;
and the pharmaceutically acceptable salts thereof and the optical isomers and distereomers thereof.

The term "5-7 membered saturated or unsaturated monocyclic heterocyclic ring having from 1 to 2 heteroatoms independently selected from N, O, and S" as defined hereinabove includes, but is not limited to, tetrahydrofuran, tetrahydrothiophene, tetramethylene sulfone, tetrahydropyran, dihydropyran, pyrrolidine, morpholine and piperidine. The term "5 to 6 membered heteroaryl" as defined hereinabove includes, but is not limited to, pyrrole, furan, thiophene, pyridine, pyrimidine, pyridazine, pyrazine,triazole, pyrazole, imidazole, isothiazole, thiazole, isoxazole and oxazole. The term "heteroaryl fused to a phenyl" includes, but is not limited to, indole, isoindole, benzofuran, benzothiophene, quinoline, isoquinoline, quinoxaline, quinazoline, benzotriazole, indazole, benzimidazole, benzothiazole, benzisoxazole, and benzoxazole.

The following compounds (**I-X**) which may be used in preparing compounds of the invention are known and references are given hereinbelow.

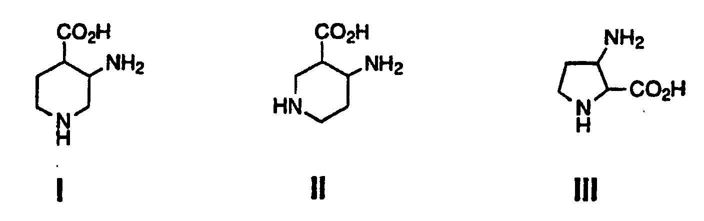

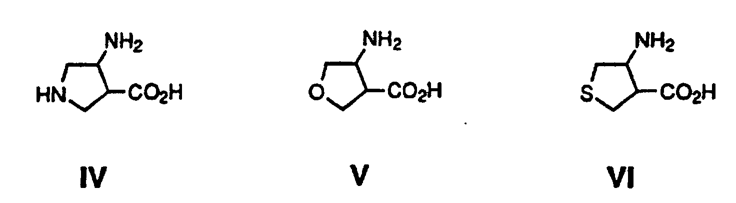

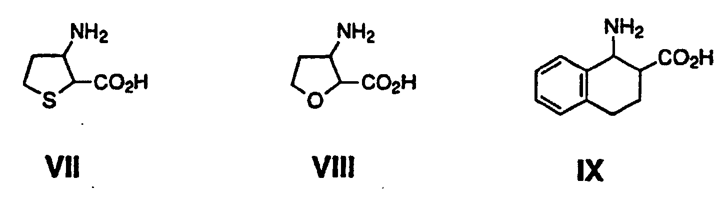

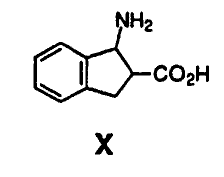

### Compounds I and II:

Jacobsen, Poul; Schaumburg, Kjeld; Krogsgaard-Larsen, Povl. **Acta Chem.**

**Scand., Ser. B** (1980), B34(5), 319-26.

### Compound III:

a) Baldwin, Jack E.; Adlington, Robert M.; Gollins, David W.; Godfrey, Christopher R. A. **Tetrahedron** (1995), 51(17), 5169-80.
b) Gallina, C.; Marta, C.; Colombo, C.; Romeo, A. **Tetrahedron** (1971), 27(19), 4681-5.
c) Kogoori, Yasushi; Wakayama, Mikio; Sano, Tetsuya; Sato, Yuji. **Jpn. Kokai Tokyo Koho**, JP 04360866 A2 .
d) Gallina, Carlo; Koch, Virginio; Romeo, Aurelio. Tetrahedron Lett. (1969), (35), 3055-6.

### Compound IV:

a) Thorbek, Pia; Hjeds, Hans; Schaumburg, Kjeld. **Acta Chem. Scand., Ser. B** (1981), B35(7), 473-9.
b) Kunisch, Franz; Mittendorf, Joachim; Plempel, Manfred; Militzer, Hans Christian. **Eur. Pat. Appl.,** EP 538692 A1.

### Compound V:

a) Kunisch, Franz; Mittendorf, Joachim; Plempel, Manfred. **Eur. Pat. Appl.,** EP 538688 A1.
b) Crowley, Patrick Jelf; Heaney, Stephen Paul; Lawson, Kevin Robert; Youle, David. **PCT Int. Appl.**, WO 9507022 Al.

### Compound VI:

a) Mittendorf, Joachim; Kunisch, Franz; Plempel, Manfred. **Eur. Pat. Appl.,** EP 538691 A1.
b) Crowley, Patrick Jelf; Heaney, Stephen Paul; Lawson, Kevin Robert; Youle, David. **PCT Int. Appl.,** WO 9507022 A1.

### Compound VII:

Mittendorf, Joachim; Kunisch, Franz; Plempel, Manfred. **Eur. Pat. Appl.,** EP 538691 A1.

### Compound VIII:

Kunisch, Franz; Mittendorf, Joachim; Plempel, Manfred. **Eur. Pat. Appl.,** EP 538688 A1.

### Compound IX:

Malis, Jerry L.; Rosenthale, Marvin E. US **Patent** 3746495.

### Compound X:

Ohki, Hidenori; Inamoto, Yoshiko; Kawabata, Kohji; Kamimura, Toshiaki; Sakane, Kazuo. **J. Antibiot.** (1991), 44(5), 546-9.

The compounds of this invention are shown to inhibit the enzymes MMP-1, MMP-9, MMP-13 and TNF-α convening enzyme (TACE) and are therefore useful in the treatment of arthritis, tumor metastasis, tissue ulceration, abnormal wound healing, periodontal disease, graft rejection, insulin resistance, bone disease and HIV infection.

### Detailed description of the Invention

The following reaction scheme (Scheme I) shows the general reaction route followed for the synthesis of hydroxamic acids of this invention. For purposes of illustration only, trans-2-aminocyclohexane carboxylic acid, wherein A is represented by a cyclohexyl ring, is sulfonylated with p-methoxybenzene sulfonamide, wherein Z is p-methoxybenzene, to provide a sulfonamide which is first convened into its t-butyl ester and then alkylated with benzyl bromide, wherein R⁷ is benzyl, to give the N,N-disubstituted sulfonamide which is subsequently converted into the corresponding hydroxamic acid in two steps.

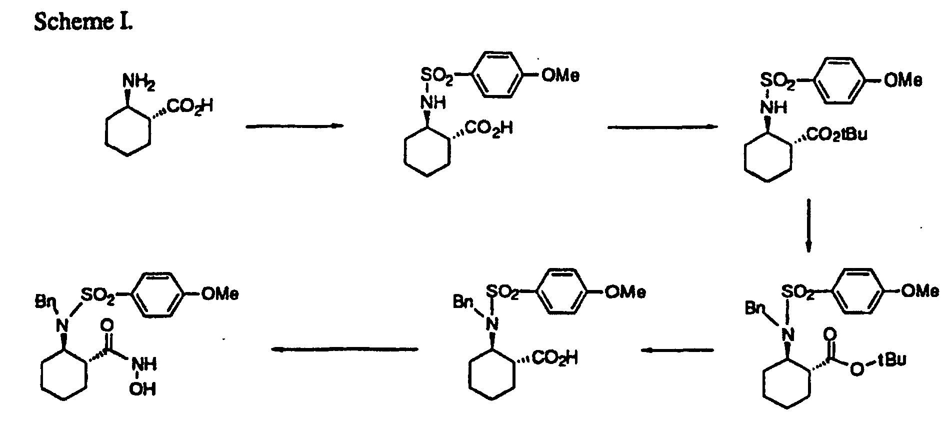

Schemes II and III illustrate two methods for incorporating amino groups into the substituent attached to the sulfonamide nitrogen of the compounds of the invention. Thus, in Scheme II the NH-sulfonamide is alkylated with propargyl bromide to provide the propargyl sulfonamide. This alkyne is reacted with paraformaldehyde in the presence of a primary or secondary amine and cuprous chloride to give the propargyl amine which is converted, as before, to the desired hydroxamic acid.

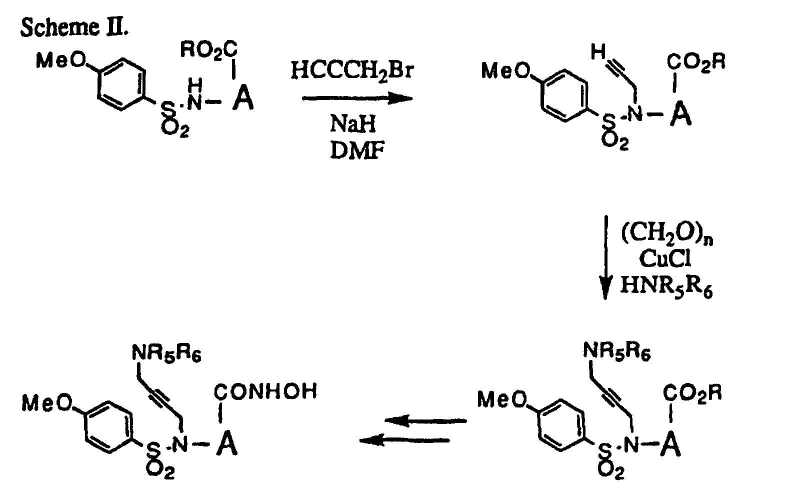

In Scheme III, selective hydrolysis of the ester of the p-carboethoxybenzyl sulfonamide group provides a mono-carboxylic acid. This acid may be converted into an amide (not shown), followed by conversion of the ester A-CO₂R into the corresponding hydroxamate, or reduced to the corresponding alcohol with diborane. The alcohol may be converted into the analogous amine via the benzylic bromide, followed by conversion of the ester A-CO₂R into the corresponding hydroxamate.

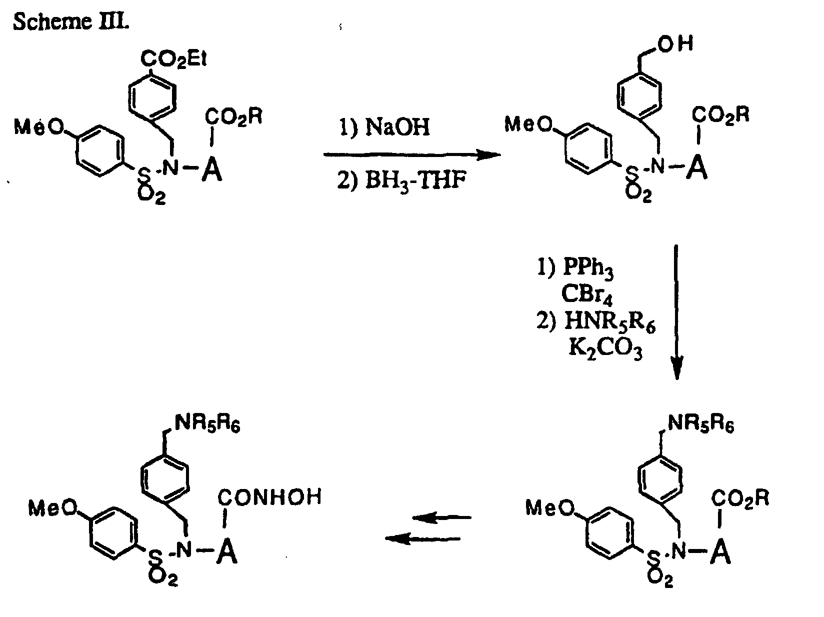

Methods for synthesizing variations of substituents on the sulfonyl aryl group are shown in Schemes IV through VI. As shown in Scheme IV, biaryl sulfonyl groups are synthesized by Suzuki couplings on a bromo-substituted benzene sulfonamide. The starting bromo-substituted benzene sulfonamide is synthesized from the commercially available bromobenzenesulfonyl chloride and the amino-acid or amino-ester, H₂N-A-CO₂R, followed by alkylation of the resulting NH-sulfonamide. Alternatively, the bromo aryl sulfonamide is converted into the corresponding boronic acid by the method of Ishiyama, et.al. [J. Org. Chem. (1995), 60, 7508] followed by coupling with an appropriate aryl halide.

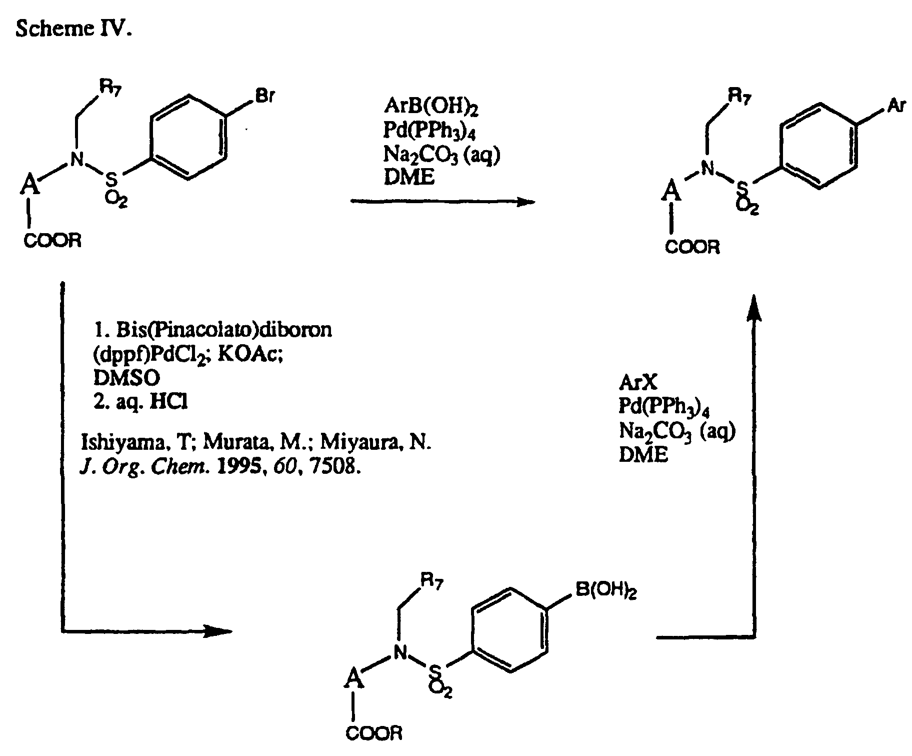

Methods for synthesizing sulfonyl aryl ethers are shown in Schemes V through VII. In Scheme V biaryl ethers, or aryl heteroaryl ethers, are synthesized starting from the known sulfonyl chlorides (see for example: Zook SE; Dagnino, R; Deason, ME, Bender, SL; Melnick, MJ WO 97/20824).

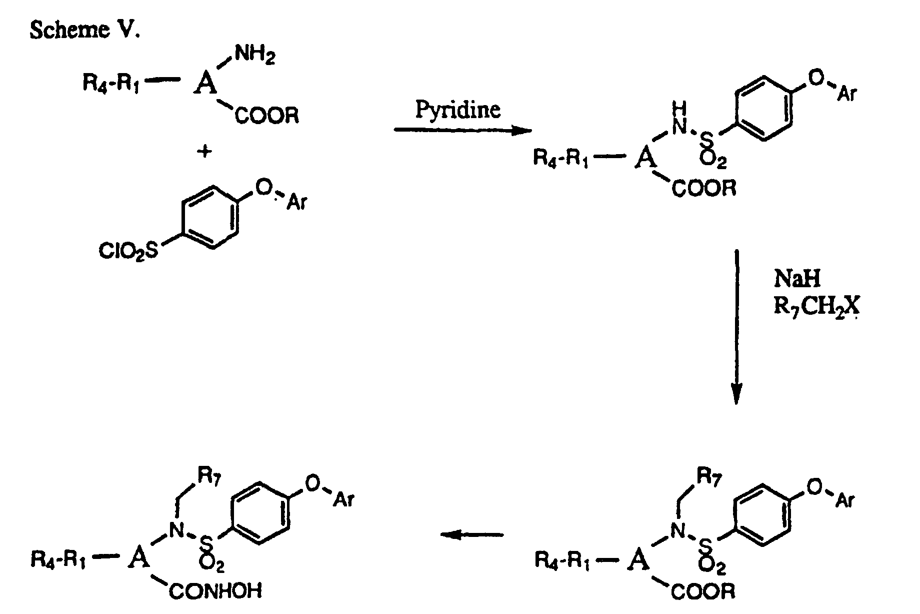

Alternatively, the biaryl ethers may be prepared from the corresponding boronic acids or via the sulfonyl phenols as shown in Scheme VI.

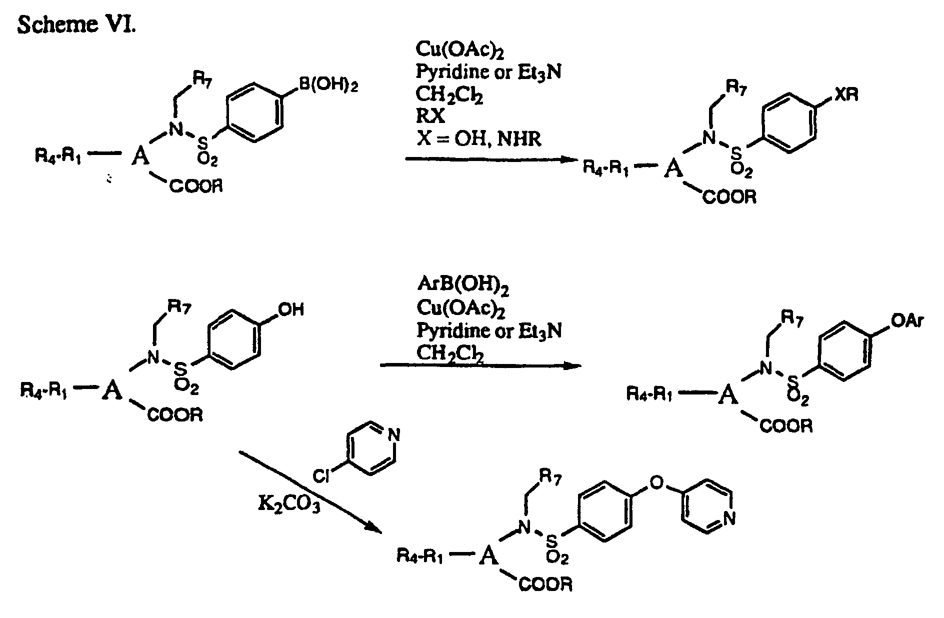

Aryl ethers may also be prepared via displacement of the fluorine from a parafluorobenzene sulfonamide, as shown in Scheme VII. Aryl or alkyl ethers may be prepared in this manner.

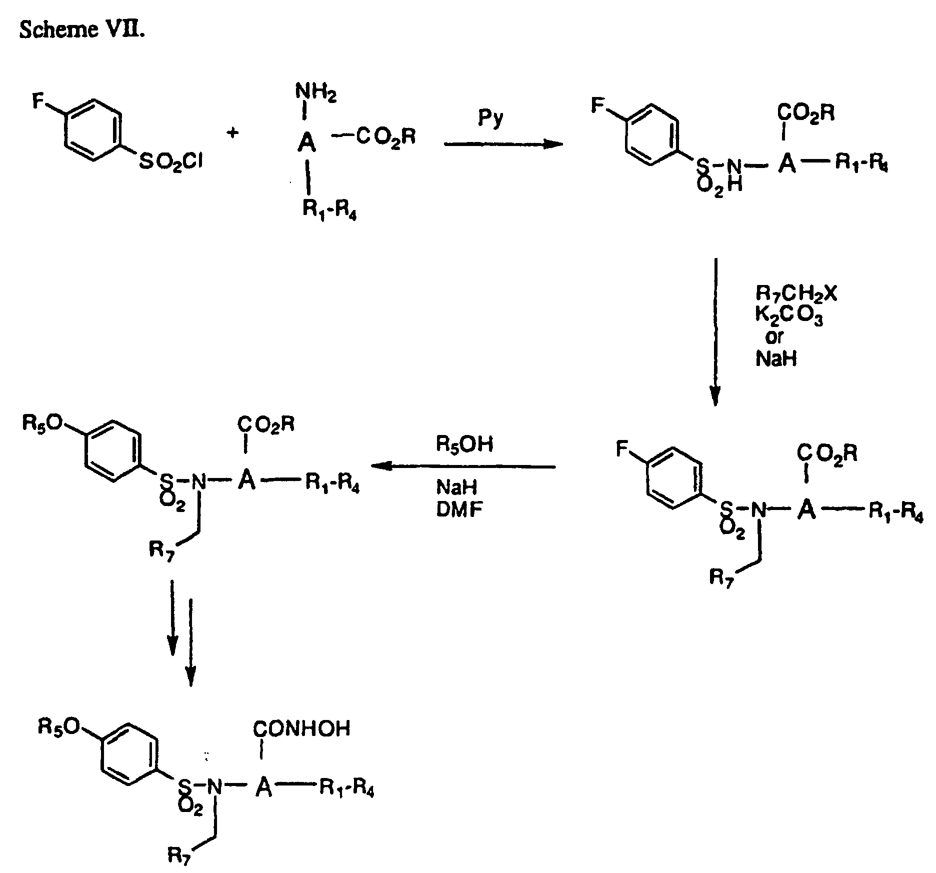

Starting materials for other groups, A, of the invention are synthetically accecssible. For example, the piperidine analogs shown in Scheme VIII should be readily available from the analogous pyridines via hydrogenation.

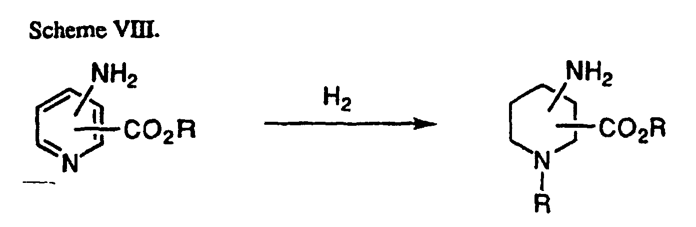

Basic salts of the hydroxamic acids can be formed with pharmaceutically acceptable alkali-forming metal cations such as lithium, sodium, potassium, calcium and aluminum. Acid addition salts can be formed when a substitutent contains a basic amino group using a pharmaceutically acceptable inorganic or organic acid such as hydrochloric, hydrobromic, phosphoric, sulfuric, acetic, benzoic, succinic, lactic, malic, maleic, fumaric or methanesulfonic acids.

The following specific examples are included for illustrative purposes and are not to be construed as limiting to this disclosure in any way. Other procedures useful for the preparation of compounds of this invention will be apparent to those skilled in the art.

### Example 1

### (trans)-2-(4-Methoxybenzenesulfonyl)aminocyclohexanecarboxylic acid

To a room temperature solution of 1g (6.8 mmol) of trans-2-amino-1-cyclohexylcarboxylic acid in 50 ml of dioxane:H₂O (1:1) containing 1.7 ml (12.2 mmol) of triethylamine was added 1.54g (7.46 mmol) of 4-methoxybenzenesulfonyl chloride. The mixture was stirred at 25 °C for 18 h. The resulting mixture was diluted with pentane to afford 1.119 g (51%) of the desired sulfonamide product as a white solid. ¹H NMR(DMSO-d₆): 7.7 ppm (dd, 2H, Ar), 7.4 ppm (d, 1H, NH), 7.0 ppm (dd, 2H, Ar), 3.8 ppm (s, 3H, OMe), 3.5 ppm (m, 1H, N-CH), 1.0-1.7 ppm (m, 9H, hydrocarbon).

### Example 2

### (cis)-2-(4-Methoxybenzenesulfonyl)aminocyclohexanecarboxylic acid

In the same manner as described in Example 1, 2.5 g (17 mmol) of cis-2-amino-1-cyclohexylcarboxylic acid provided 3.283 g (60%) of the desired carboxylic acid. Electrospray Mass Spec 314.1 (M+H).

### Example 3

### (trans)-2-(4-Methoxybenzenesulfonyl)aminocyclohexanecarboxylic acid t-butyl ester

To a solution of 0.313g (1 mmol) of the product from Example 1 in 5.0 mL of toluene was added 1 mL (4 mmol) of N,N-dimethylformamide di-tert-butyl acetal. The resulting mixture was heated at 110 °C for 4h under nitrogen and then allowed to cool to room temperature. The solution was then poured on top of a silica gel column. Chromatography on silica gel eluting with 10-20% ethyl acetate/hexane gave 353 mg (96%) of the desired ester as a white solid. ¹H NMR(CDCl₃): 7.8 ppm (dd, 2H, Ar), 7.0 ppm (dd, 2H, Ar), 5.7 ppm (d, 1H, NH), 3.9 ppm (s, 3H, OMe), 3.4 ppm (m, 1H, N-CH), 2.5 ppm (m, 1H, CH-CO₂-), 1.0-2.0 ppm (m, 17H, hydrocarbon).

### Example 4

### (cis)-2-(4-Methoxy-benzenesulfonylamino)-cyclohexanecarboxylic acid tert-butyl ester

In the same manner as described in Example 3, 1.438 g (4.59 mmol) of the product from Example 2 provided 0.739 g (44%) of the desired tert-butyl ester as a colorless oil. Electrospray Mass Spec 370.1 (M+H).

### Example 5

### (trans)-2-[Benzyl-(4-methoxybenzenesulfonyl)amino]-cyclohexanecarboxylic acid t-butyl ester

To a solution of 1.146 g (3.1 mmol) of the product from Example 3 in 31 mL of DMF was added 0.137g (3.42 mmol) of 60% sodium hydride. The resulting mixture was stirred for 30 min at 25°C and then 0.42 mL (3.50 mmol) of benzyl bromide was added all at once. This reaction mixture was stirred for 10 hr at 55 °C and then poured into water and extracted with ether. The combined organics were washed with water and brine, dried over MgSO₄, filtered and concentrated in vacuo to provide a white solid which was recrystallized from EtOAc/Hexanes to provide 1.364 g (95%) of the desired product. ¹H NMR(CDCl₃): 7.7 ppm (dd, 2H, Ar), 7.1-7.4 (m, 5H, Ar), 6.9 ppm (dd, 2H, Ar), 4.5-4.7 ppm (AB quartet, 2H, CH₂-Ar), 3.9 ppm (s, 3H, OMe), 4.0 ppm (m, 1H, N-CH), 2.9 ppm (m, 1H, CH-CO₂-), 1.0-2.3 ppm (m, 17H, hydrocarbon protons).

### Example 6

### (cis)-2-[Benzyl-(4-methoxy-benzenesulfonyl)-amino]-cyclohexanecarboxylic acid tert-butyl ester

In the same manner as described in Example 5, 0.600 g (1.62 mmol) of the product from Example 4 provided 0.310 g (42%) of the desired benzylated ester as a colorless oil. Electrospray Mass Spec 460.1 (M+H).

### Example 7

### (trans)-2-[Benzyl-(4-methoxy-benzenesulfonyl)-amino]-cyclohexanecarboxylic acid

To a solution of 1.364 g (2.97 mmol) of the product from Example 5 in 10mL of dichloromethane was added 10mL of trifluoroacetic acid and the mixture was stirred for 4h at room temperature. The solvent was then concentrated in vacuo and the residue was chromatographed on silica gel eluting with 10-100% ethyl acetate/hexane to provide 1.092 g (73%) of the desired product as a white solid. Electrospray Mass Spec 404.2 (M+H)

### Example 8

### (cis)-2-[Benzyl-(4-methoxy-benzenesulfonyl)-amino]-cyclohexanecarboxylic acid

In the same manner as described in Example 7, 0.240 g (0.522 mmol) of the product from Example 6 provided 0.207 g (98%) of the desired carboxylic acid as a white solid. Electrospray Mass Spec 404.0 (M-H).

### Example 9

### (trans)-2-[Benzyl-(4-methoxy-benzenesulfonyl)-amino]-cyclohexanecarboxylic acid hydroxyamide

To a solution of 807 mg (2 mmol) of the product from Example 7 in 20 mL of dichloromethane was added 0.05 mL of DMF followed by 2.2 mL (2.52 mmol) of a 2 M solution of oxalyl chloride and the resulting reaction mixture was stirred at room temperature for 0.5 h.

In a separate flask, 4 mL (29 mmol) of triethylamine was added to a 0°C mixture of 695 mg (10 mmol) of hydroxylamine hydrochloride in 22 mL of THF and 5 mL of water. After this mixture had been stirred for 15min at 0 °C, the acid chloride solution was added to it in one portion and the resulting solution was allowed to warm to room temperature and stirred for another 4h. Water was then added to the reaction flask and 854 mg (100% yield) of the desired hydroxamic acid was collected via filtration as a white solid. Electrospray Mass Spec 419.3 (M+H)

### Example 10

### (cis)-2-[Benzyl-(4-methoxy-benzenesulfonyl)-amino]-cyclohexanecarboxylic acid hydroxyamide

In the same manner as described in Example 9, 0.144 g (0.357 mmol) of the product from Example 8 provided 0.90 g (60%) of the desired carboxylic acid as a white solid. Electrospray Mass Spec 419.1 (M+H).

### Pharmacology

### Procedures for Measuring MMP-1, MMP-9, and MMP-13 Inhibition

These assays are based on the cleavage of a thiopeptide substrates such as Ac-Pro-Leu-Gly(2-mercapto-4-methyl-pentanoyl)-Leu-Gly-OEt by the matrix metalloproteinases MMP-1, MMP-13 (collagenases) or MMP-9 (gelatinase), which results in the release of a substrate product that reacts colorimetrically with DTNB (5,5'-dithiobis(2-nitro-benzoic acid)). The enzyme activity is measured by the rate of the color increase. The thiopeptide substrate is made up fresh as a 20 mM stock in 100% DMSO and the DTNB is dissolved in 100% DMSO as a 100 mM stock and stored in the dark at room temperature. Both the substrate and DTNB are diluted together to 1 mM with substrate buffer (50 mM HEPES pH 7.5, 5 mM CaCl₂) before use. The stock of enzyme is diluted with assay buffer (50 mM HEPES, pH 7.5, 5 mM CaCl₂, 0.02% Brij) to the desired final concentration. The assay buffer, enzyme, vehicle or inhibitor, and DTNB/substrate are added in this order to a 96 well plate (total reaction volume of 200 µl) and the increase in color is monitored spectrophotometrically for 5 minutes at 405 nm on a plate reader and the increase in color over time is plotted as a linear line.

Alternatively, a fluorescent peptide substrate is used. In this assay, the peptide substrate contains a fluorescent group and a quenching group. Upon cleavage of the substrate by an MMP, the fluorescence that is generated is quantitated on the fluorescence plate reader. The assay is run in HCBC assay buffer (50mM HEPES, pH 7.0, 5 mM Ca⁺², 0.02% Brij, 0.5% Cysteine), with human recombinant MMP-1, MMP-9, or MMP-13. The substrate is dissolved in methanol and stored frozen in 1 mM aliquots. For the assay, substrate and enzymes are diluted in HCBC buffer to the desired concentrations. Compounds are added to the 96 well plate containing enzyme and the reaction is started by the addition of substrate. The reaction is read (excitation 340 nm, emission 444 nm) for 10 min, and the increase in fluorescence over time is plotted as a linear line.

For either the thiopeptide or fluorescent peptide assays, the slope of the line is calculated and represents the reaction rate. The linearity of the reaction rate is confirmed (r² >0.85). The mean (x±sem) of the control rate is calculated and compared for statistical significance (p<0.05) with drug-treated rates using Dunnett's multiple comparison test. Dose-response relationships can be generated using multiple doses of drug and IC₅₀ values with 95% CI are estimated using linear regression.

### Procedure for Measuring TACE Inhibition

Using 96-well black microtiter plates, each well receives a solution composed of 10 µL TACE (Immunex, final concentration 1µg/mL), 70µL Tris buffer, pH 7.4 containing 10% glycerol (final concentration 10 mM), and 10 µL of test compound solution in DMSO (final concentration 1µM, DMSO concentration <1%) and incubated for 10 minutes at room temperature. The reaction is initiated by addition of a fluorescent peptidyl substrate (final concentration 100 µM) to each well and then shaking on a shaker for 5 sec.

The reaction is read (excitation 340 nm, emission 420 nm) for 10 min. and the increase in fluorescence over time is plotted as a linear line. The slope of the line is calculated and represents the reaction rate.

The linearity of the reaction rate is confirmed (r² >0.85). The mean (x±sem) of the control rate is calculated and compared for statistical significance (p<0.05) with drug-treated rates using Dunnett's multiple comparison test. Dose-response relationships can be generate using multiple doses of drug and IC₅₀ values with 95% CI are estimated using linear regression.

Results of the above in-vitro matrix metalloproteinase inhibition and TACE inhibition pharmacological assays are given in Table I below.

**Table I.**

| Inhibition of MMP and TACE | | | | |
|---|---|---|---|---|
| Example | MMP-1¹ | MMP-9¹ | MMP-13¹ | TACE¹ |
| 9 | 176 | 181 | 233 | 1612 |
| 10 | 616 | 275 | 286 | 24% |

| | | | | |
|---|---|---|---|---|
| 1. IC₅₀nM or % inhibition at 1 µM concentration | | | | |

### Pharmaceutical Composition

Compounds of this invention may be administered neat or with a pharmaceutical carrier to a patient in need thereof. The pharmaceutical carrier may be solid or liquid.

Applicable solid carriers can include one or more substances which may also act as flavoring agents, lubricants, solubilizers, suspending agents, fillers, glidants, compression aids, binders or tablet-disintegrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such a solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above, e.g., cellulose derivatives, preferable sodium carboxymethyl cellulose solution), alcohols (including monohydric alcohols and polyhydric alcohols, e.g., glycols) and their derivatives, and oils (e.g., fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

The compounds of this invention may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non-toxic to the skin, and allows delivery of the agent for systemic absorption into the blood stream via the skin. The carrier may take any number of forms such as creams and ointments, pastes, gels, and occlusive devices. The creams and ointments may be viscous liquid or semi-solid emulsions of either the oil in water or water in oil type. Pastes comprised of absorptive powders dispersed in petroleum or hydrophilic petroleum containing the active ingredient may also be suitable. A variety of occlusive devices may be used to release the active ingredient into the blood stream such as a semipermeable membrane covering a reservoir containing the active ingredient with or without a carrier, or a matrix containing the active ingredient. Other occlusive devices are known in the literature.

The dosage to be used in the treatment of a specific patient suffering a MMP or TACE dependent condition must be subjectively determined by the attending physician. The variables involved include the severity of the dysfunction, and the size, age, and response pattern of the patient. Treatment will generally be initiated with small dosages less than the optimum dose of the compound. Thereafter the dosage is increased until the optimum effect under the circumstances is reached. Precise dosages for oral, parenteral, nasal, or intrabronchial administration will be determined by the administering physician based on experience with the individual subject treated and standard medical principles.

Preferably the pharmaceutical composition is in unit dosage form, e.g., as tablets or capsules. In such form, the composition is sub-divided in unit dose containing appropriate quantities of the active ingredient; the unit dosage form can be packaged compositions, for example packed powders, vials, ampoules, prefilled syringes or sachets containing liquids. The unit dosage form can be, for example, a capsule or tablet itself, or it can be the appropriate number of any such compositions in package form.

## Claims

1. A compound having the formula: 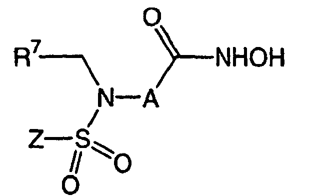 where the hydroxamic acid moiety and the sulfonamide moiety are bonded to adjacent carbons of group A where:
A is a 5 to 7 membered, monocyclic, non-aromatic heterocyclic ring having from 1 to 2 heteroatoms independently selected from N, O, and S, optionally substituted by R¹, R², R³ and R⁴;
a -C₃-C₇-cycloalkyl containing 0-2 double bonds and optionally substituted with R¹, R², R³ and R⁴;
or -CHR⁵=CHR⁶-;
Z is aryl, heteroaryl, or heteroaryl fused to a phenyl,
where aryl is phenyl or naphthyl optionally substituted by R¹, R², R³ and R⁴;
heteroaryl is a 5-6 membered heteroaromatic ring having from 1 to 3 heteroatoms independently selected from N, O, and S, and optionally substituted by R¹, R², R³ and R⁴;
and when heteroaryl is fused to phenyl, either or both of the rings can be optionally substituted by R¹, R², R³ and R⁴;
R¹, R², R³ and R⁴ are independently -H, -COR⁵, -F,-Br, -Cl, -I,
-C(O)NR⁵OR⁶,-CN, -OR⁵,-C₁-C₄-perfluoroalkyl, -S(O)ₓR⁵ where x is 0-2, -OPO(OR⁵)OR⁶, -PO(OR⁶)R⁵, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, -NR⁵C(=NR⁶)NR⁵R⁶, 3-6 membered
cycloheteroalkyl having one to three heteroatoms independently selected from N, O, and S, optionally having 1 or 2 double bonds and optionally substituted by one to three groups each selected independently from R⁵; -aryl or heteroaryl as defined above, -SO₂NHCOR⁵ or -CONHSO₂R⁵ where R⁵ is not H, -tetrazol-5-yl, -SO₂NHCN, -SO₂NHCONR⁵R⁶ or straight chain or branched -C₁-C₆ alkyl, -C₂-C₆-alkenyl, -C₂-C₆-alkynyl or C₃-C₆cycloalkyl optionally having 1 or 2 double bonds each optionally substituted with
-COR⁵, -CN, -C₂-C₆ alkenyl, -C₂-C₆ alkynyl,-OR⁵, -C₁-C₄-perfluoroalkyl, -S(O)ₓR⁵ where x is 0-2, -OC(O)NR⁵R⁶, -COOR⁵, -CONR⁵R⁶, -SO₃H, -NR⁵R⁶,-NR⁵COR⁶, -NR⁵COOR⁶, -SO₂NR⁵R⁶, -NO₂, -N(R⁵)SO₂R⁶, -NR⁵CONR⁵R⁶, -C₃-C₆ cycloalkyl as defined above, 3-6 membered cycloheteroalkyl as defined above, aryl or heteroaryl as defined above, -SO₂NHCOR⁵ or-CONHSO₂R⁵ where R⁵ is not hydrogen, -PO(OR⁵)OR⁶, -PO(OR⁶)R⁵, -tetrazol-5-yl, -C(O)NR⁵OR⁶, -NR⁵C(=NR⁶)NR⁵R⁶,-SO₂NHCONR⁵R⁶ or -SO₂NHCN;
with the proviso that when R¹ and R² are on adjacent carbons of A, R¹ and R² together with the carbons to which they are attached can form a 5-7 membered saturated or unsaturated non-aromatic monocyclic heterocyclic ring, or a 5-6 membered heteroaryl ring, each having from 1 to 2 heteroatoms independently selected from N, O, and S, wherein said heterocyclic or heteroaryl ring may be optionally substituted by one to four groups each selected independently from R⁴; or R¹ and R² together with the carbons to which they are attached can form a 5-7 membered saturated or unsaturated carbocyclic ring or an aryl ring wherein said carbocyclic or aryl ring may be optionally substituted by one to four groups each selected independently from R4;
R⁵ and R⁶ are independently defined as H, aryl and heteroaryl as defined above, -C₃-C₆-cycloalkyl as defined above, -C₃-C₆-cycloheteroalkyl as defined above, -C₁-C₄-perfluoroalkyl, or straight chain or branched -C₁-C₆ alkyl, -C₂-C₆-alkenyl, or -C₂-C₆-alkynyl each optionally substituted with -OH,
-COR⁸, -CN, -C(O)NR⁸OR⁹, -C₂-C₆-alkenyl, -C₂-C₆-alkynyl, -OR⁸, -C₁-C₄-perfluoroalkyl, -S(O)ₓR⁸ where x is 0-2, -OPO(OR⁸)OR⁹, -PO(OR⁸)R⁹, -OC(O)NR⁸R⁹, -COOR⁸,--CONR⁸R⁹, -SO₃H, -NR⁸R⁹,-NCOR⁸R⁹, -NR⁸COOR⁹, -SO₂NR⁸R⁹, -NO₂, -N(R⁸)SO₂R⁹, -NR⁸CONR⁸R⁹, -C₃-C₆ cycloalkyl as defined above, -C₃-C₆- cycloheteroalkyl as defined above, -aryl or heteroaryl as defined above, -SO₂NHCOR⁸ or -CONHSO₂R⁸ where R⁸ is not hyrdogen, -tetrazol-5-yl, -NR⁸C(=NR⁹)NR⁸R⁹, -SO₂NHCONR⁸R⁹, -SO₂NHCN;
R⁷ is hydrogen, straight chain or branched -C₁-C₆-alkyl, -C₂-C₆-alkenyl, or -C₂-C₆-alkynyl each optionally substituted with -OH, -COR⁵, -CN, -C₂-C₆-alkenyl, -C₂-C₆-alkynyl, -OR⁵, -C₁-C₄-perfluoroalkyl, -S(O)ₓR⁵ where x is 0-2, -OPO(OR⁵)OR⁶, -PO(OR⁵)R⁶, -OC(O)NR⁵R⁶, -COOR⁵, -CONR⁵R⁶, -SO₃H, -NR⁵R⁶,-NR⁵COR⁶, -NR⁵COOR⁶, -SO₂NR⁵R⁶, -NO₂, -N(R⁵)SO₂R⁶, -NR⁵CONR⁵R⁶, -C₃-C₆ cycloalkyl as defined above, -C₃-C₆-cycloheteroalkyl as defined above, -aryl or heteroaryl as defined above, -SO₂NHCOR⁵ or -CONHSO₂R⁵ where R⁵ is not hydrogen, -tetrazol-5-yl, -NR⁵C(=NR6)NR⁵R⁶, -C(O)N R⁵OR⁶, -SO₂NHCONR⁵R⁶ or -SO₂NHCN;
or R⁷ is phenyl or naphthyl, optionally substituted by R¹, R², R³ and R⁴ or a 5 to 6 membered heteroaryl group having 1 to 3 heteroatoms selected independently from N, O, and S and optionally substituted by R¹, R², R³ and R⁴;
or R⁷ is C₃-C₆ cycloalkyl or 3-6 membered cycloheteroalkyl as defined above;
or R⁷CH₂-N-A-, where A is as defined above, can form with a carbon atom of A adjacent to the carbon bearing the sulfonamido group, a non-aromatic fused 7-10 membered heterocyclic ring optionally containing an additional heteroatom selected from O, S and N wherein said heterocyclic ring may be optionally fused to a benzene ring;
R⁸ and R⁹ are independently H, aryl or heteroaryl as defined above, -C₃-C₇-cycloalkyl or cycloheteroalkyl as defined above, -C₁-C₄-perfluoroalkyl, straight chain or branched -C₁-C₆-alkyl, -C₂-C₆-alkenyl, or -C₂-C₆-alkynyl, each optionally substituted with hydroxy, alkoxy, aryloxy, -C₁-C₄-perfluoroalkyl, amino, mono- and di-C₁-C₆-alkylamino, carboxylic acid, carboalkoxy and carboaryloxy, nitro, cyano, carboxamido primary, mono- and di-C₁-C₆-alkylcarbamoyl;
an optical isomer or diastereomer thereof;
or a pharmaceutically acceptable salt thereof.

2. A compound according to claim 1 wherein the Z group is para-alkoxyphenyl, paraaryloxyphenyl or para-heteroaryloxyphenyl.

3. A compound according to claim 2 which is selected from the group consisting of:
(trans)-2-[Benzyl-(4-methoxy-benzenesulfonyl)-amino]-cyclohexanecarboxylic acid hydroxyamide, and
(cis)-2-[Benzyl-(4-methoxy-benzenesulfonyl)-amino]-cyclohexanecarboxylic acid hydroxyamide.

4. Use of a compound as claimed in any one of claims 1 to 3 in the preparation of a medicament for inhibiting pathological changes in a mammal mediated by matrix metalloproteinases

5. A use according to claim 4 wherein the condition treated is atherosclerosis, atherosclerotic plaque formation, reduction of coronary thrombosis from atherosclerotic plaque rupture, restenosis, MMP-mediated osteopenias, inflammatory diseases of the central nervous system, skin aging, angiogenesis, tumor metastasis, tumor growth, osteoarthritis, rheumatoid arthritis, septic arthritis, corneal ulceration, abnormal wound healing, bone disease, proteinuria, aneurysmal aortic disease, degenerative cartilage loss following traumatic joint injury, demyelinating diseases of the nervous system, cirrhosis of the liver, glomerular disease of the kidney, premature rupture of fetal membranes, infammatory bowel disease, or periodontal disease.

6. A use according to claim 4 wherein the condition treated is age related macular degeneration, diabetic retinopathy, proliferative vitreoretinopathy, retinopathy of prematurity, ocular inflammation, keratoconus, Sjogren's syndrome, myopia, ocular tumors, ocular angiogenesis/neovascularization and corneal graft rejection.

7. Use of a compound as claimed in any one of claims 1 to 3 in the preparation of a medicament for inhibiting pathological changes in a mammal mediated by TNF-α converting enzyme (TACE).

8. A use according to claim 7 wherein the condition treated is rheumatoid arthritis, graft rejection, cachexia, anorexia, inflammation, fever, insulin resistance, septic shock, congestive heart failure, inflammatory disease of the central nervous system, inflammatory bowel disease, or HIV infection.

9. A pharmaceutical composition comprising a pharmaceutical carrier and a matrix metalloproteinase or TACE inhibiting compound according to claim 1.

10. A pharmaceutical composition comprising a pharmaceutical carrier and a compound according to any one of claims 1 to 3.

11. A compound as claimed in any one of claims 1 to 3 for use as a medicament.

12. A method for the preparation of a compound as claimed in Claim 1 which comprises reacting a compound of formula: 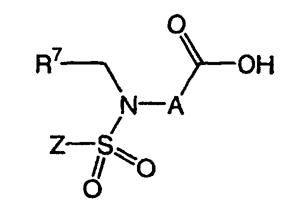 wherein Z, A and R⁷ are as defined in Claim 1 with hydroxylamine.

## Patentansprüche

1. Verbindung mit der Formel: 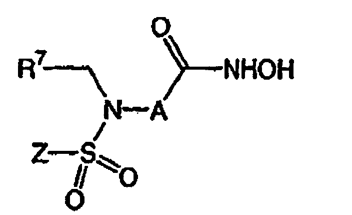 worin die Hydroxamsäurekomponente und die Sulfonamidokomponente an nebeneinanderliegende Kohlenstoffe von Gruppe A gebunden sind, worin:
A einen 5- bis 7-gliedrigen, monocyclischen, nicht aromatischen, heterocyclischen Ring mit von 1 bis 2 Heteroatomen, unabhängig ausgewählt aus N, O und S, gegebenenfalls substituiert durch R¹, R², R³ und R⁴;
ein -C₃-C₇-cycloalkyl, enthaltend 0-2 Doppelbindungen und gegebenenfalls substituiert mit R¹, R², R³ und R⁴;
oder -CHR⁵=CHR⁶- darstellt;
Z Aryl, Heteroaryl oder an ein Phenyl kondensiertes Heteroaryl darstellt,
worin Aryl für Phenyl oder Naphthyl steht, gegebenenfalls substituiert durch R¹, R², R³ und R⁴;
Heteroaryl für einen 5-6-gliedrigen, heteroaromatischen Ring mit von 1 bis 3 Heteroatomen, unabhängig ausgewählt aus N, O und S steht, und gegebenenfalls substituiert durch R¹, R², R³ und R⁴;
und wenn Heteroaryl an Phenyl kondensiert ist, können entweder einer oder beide Ringe gegebenenfalls durch R¹, R², R³ und R⁴ substituiert sein;
R¹, R², R³ und R⁴ unabhängig H, -COR⁵, -F, -Br, -Cl, -I, -C(O)NR⁵OR⁶, -CN, -OR⁵, -C₁-C₄-perfluoralkyl, -S(O)ₓR⁵, worin x 0-2 ist, -OPO(OR⁵)OR⁶, -PO(OR⁶)R⁵, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, -NR⁵C(=NR⁶)NR⁵R⁶, 3-6-gliedriges Cycloheteroalkyl mit ein bis drei Heteroatomen, unabhängig ausgewählt aus N, O und S, gegebenenfalls mit 1 oder 2 Doppelbindungen und gegebenenfalls substituiert durch eine bis drei Gruppen, jede unabhängig ausgewählt aus R⁵, -aryl oder -heteroaryl, wie oben definiert, -SO₂NHCOR⁵ oder -CONHSO₂R⁵ worin R⁵ nicht für H steht, -tetrazol-5-yl, -SO₂NHCN, -SO₂NHCONR⁵R⁶ oder gradkettiges oder verzweigtes -C₁-C₆alkyl, -C₂-C₆-alkenyl, -C₂-C₆-alkinyl oder C₃-C₆-cycloalkyl, gegebenenfalls mit 1 oder zwei Doppelbindungen darstellen, jedes gegebenenfalls substituiert mit
-COR⁵, -CN, -C₂-C₆-alkenyl, -C₂-C₆-alkinyl, -OR⁵, -C₁-C₄perfluoralkyl, -S(O)ₓR⁵ worin x 0-2 ist, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, -C₃-C₆-cycloalkyl wie oben definiert, 3-6-gliedriges Cycloalkyl wie oben definiert, Aryl oder Heteroaryl wie oben definiert, -SO₂NHCOOR⁶ oder -CONHSO₂R⁵ worin R⁵ nicht für Wasserstoff steht, -PO(OR⁵)OR⁶, -PO(OR⁶)R⁵, -tetrazol-5-yl, -C(O)NR⁵OR⁶, -NR⁵C(=NR⁶)NR⁵R⁶, -SO₃NHCONR⁵R⁶ oder -SO₂NHCN;
unter der Bedingung, dass wenn sich R¹ und R² an nebeneinanderliegenden Kohlenstoffen von A befinden, R¹ und R² zusammen mit den Kohlenstoffen, an welche sie gebunden sind, einen 5-7-gliedrigen, gesättigten oder ungesättigten, nicht aromatischen, monocyclischen, heterocyclischen Ring oder einen 5-6-gliedrigen Heteroarylring bilden können, jeder mit von 1 bis 2 Heteroatomen, unabhängig ausgewählt aus N, O und S, worin besagter heterocyclischer oder Heteroarylring gegebenenfalls substituiert sein kann durch ein bis vier Gruppen, jede unabhängig ausgewählt aus R⁴; oder R¹ und R² zusammen mit den Kohlenstoffen, an welche sie gebunden sind, einen 5-7-gliedrigen, gesättigten oder ungesättigten, carbocyclischen Ring bilden können, oder einen Arylring, wobei besagter carbocyclischer oder Arylring gegebenenfalls substituiert sein kann durch eine bis vier Gruppen, jede unabhängig ausgewählt aus R⁴;
R⁵ und R⁶ unabhängig definiert sind als H, Aryl und Heteroaryl, wie oben definiert, -C₃-C₆-cycloalkyl wie oben definiert, -C₃-C₆-cycloheteroalkyl wie oben definiert, -C₁-C₄-perfluoralkyl oder gradkettiges oder verzweigtes -C₁-C₆-alkyl, -C₂-C₆-alkenyl oder -C₂-C₆-alkinyl, jedes gegebenenfalls substituiert mit -OH, -COR⁸, -CN, -C(O)NR⁸OR⁹, -C₂-C₆-alkenyl, -C₂-C₆-alkinyl, -OR⁸, -C₁-C₄-perfluoralkyl, -S(O)ₓR⁸ worin x 0-2 ist, -OPO(OR⁸)OR⁹, -PO(OR⁸)R⁹, -OC(O)NR⁸R⁹, COOR⁸, -CONR⁸R⁹, -SO₃H, -NR⁸R⁹, NCOR⁸R⁹, -NR⁸COOR⁹, -SO₂NR⁸R⁹, -NO₂, -N(R⁸)SO₂R⁹, -NR⁸CONR⁸R⁹, -C₃-C₆-cycloalkyl wie oben definiert, -C₃-C₆cycloheteroalkyl wie oben definiert, -aryl oder -heteroaryl wie oben definiert, -SO₂NHCOR⁸ oder -CONHSO₂R⁸, worin R⁸ nicht für Wasserstoff steht, -tetrazol-5-yl, -NR⁸C(=NR⁹)NR⁸R⁹, -SO₂NHCONR⁸R⁹, -SO₂NHCN;
R⁷ Wasserstoff, gradkettiges oder verzweigtes -C₁-C₆-alkyl, -C₂-C₆-alkenyl oder -C₂-C₆-alkinyl darstellt, jedes gegebenenfalls substituiert mit -OH, -COR⁵, -CN, -C₂-C₆-alkenyl, -C₂-C₆-alkinyl, -OR⁵, -C₁-C₄-perfluoralkyl, -S(O)ₓR⁵ worin x 0-2 ist, -OPO(OR⁵)OR⁶, -PO(OR⁵)R⁶, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, -C₃-C₆-cycloalkyl wie oben definiert, -C₃-C₆cycloheteroalkyl wie oben definiert, -aryl oder -heteroaryl wie oben definiert, -SO₂NHCOR⁵ oder -CONHSO₂R⁵, worin R⁵ nicht für Wasserstoff steht, -tetrazol-5-yl, -NR⁵C(=NR⁶)NR⁵R⁶, -C(O)NR⁵OR⁶, -SO₂NHCONR⁵R⁶ oder -SO₂NHCN;
oder R⁷ Phenyl oder Naphthyl darstellt, gegebenenfalls substituiert durch R¹, R², R³ und R⁴ oder eine 5- bis 6-gliedrige Heteroarylgruppe mit 1 bis 3 Heteroatomen, unabhängig ausgewählt aus N, O und S und gegebenenfalls substituiert durch R¹, R², R³ und R⁴;
oder R⁷ für C₃-C₆-cycloalkyl oder 3-6-gliedriges Cycloheteroalkyl steht, wie oben definiert;
oder R⁷CH₂-N-A, worin A wie oben definiert ist, mit einem Kohlenstoffatom von A, welches an den Kohlenstoff angrenzt, welcher die Sulfonamidogruppe trägt, einen nicht aromatischen, kondensierten, 7-10-gliedrigen, heterocyclischen Ring bilden kann, welcher gegebenenfalls ein zusätzliches Heteroatom enthält, ausgewählt aus 0, S und N, worin besagter heterocyclischer Ring gegebenenfalls an einen Benzolring kondensiert sein kann;
R⁸ und R⁹ unabhängig H, Aryl oder Heteroaryl wie oben definiert, -C₃-C₇-cycloalkyl oder -cycloheteroalkyl wie oben definiert, -C₁-C₄-perfluoralkyl, gradkettiges oder verzweigtes -C₁-C₆alkyl, -C₂-C₆-alkenyl oder C₂-C₆-alkinyl, jedes gegebenenfalls substituiert mit Hydroxy, Alkoxy, Aryloxy, -C₁-C₄perfluoralkyl, Amino, Mono- und Di-C₁-C₆-alkylamino, Carbonsäure, Carboalkoxy und Carboaryloxy, Nitro, Cyano, Carboxamido primär, Mono- und Di-C₁-C₆-alkylcarbomyl darstellen;
ein optisches Isomer oder Diastereomer davon;
oder ein pharmazeutisch annehmbares Salz davon.

2. Verbindung gemäß Anspruch 1, worin die Z-Gruppe para-Alkoxyphenyl, para-Aryloxyphenyl oder para-Heteroaryloxyphenyl darstellt.

3. Verbindung gemäß Anspruch 2, welche ausgewählt wird aus der Gruppe bestehend aus:
(trans)-2-[Benzyl-(4-methoxy-benzolsulfonyl)-amino]-cyclohexancarbonsäure-hydroxyamid und
(cis)- 2-[Benzyl-(4-methoxy-benzolsulfonyl)-amino]-cyclohexancarbonsäure-hydroxyamid.

4. Verwendung einer Verbindung, wie in einem der Ansprüche 1 bis 3 beansprucht, bei der Herstellung eines Arzneimittels zum Hemmen pathologischer Veränderungen in einem Säuger, vermittelt durch Matrix-Metalloproteinasen.

5. Verwendung gemäß Anspruch 4, wobei der behandelte Zustand Atherosklerose, atherosklerotische Plaque-Bildung, Reduktion koronarer Thrombose von atherosklerotischem Plaque-Riss, Restenose, MMP-vermittelte Osteopenien, Entzündungserkrankungen des zentralen Nervensystems, Hautalterung, Angiogenese, Tumormetastase, Tumorwachstum, Osteoarthritis, Rheumatoidarthritis, septische Arthritis, korneale Geschwürbildung, abnorme Wundheilung, Knochenerkrankung, Proteinurie, aneurysmatische Aortaerkrankung, degenerativer Knorpelverlust nach traumatischer Gelenkverletzung, demyelinisierende Erkrankungen des Nervensystems, Leberzirrhose, glomerulare Erkrankung der Nieren, vorzeitiger Riss fötaler Membranen, entzündliche Darmerkrankung oder periodontale Erkrankung ist.

6. Verwendung gemäß Anspruch 4, wobei der behandelte Zustand altersbezogene Makuladegeneration, diabetische Retinopathie, proliferative Vitreoretinopathie, Frühgeborenenretinopathie, okulare Entzündung, Keratoconus, Sjögren-Syndrom, Myopie, okulare Tumore, okulare Angiogenese/Neovaskularisation und korneale Transplantatabstoßung ist.

7. Verwendung einer Verbindung, wie in einem der Ansprüche 1 bis 3 beansprucht, bei der Herstellung eines Arzneimittels zum Hemmen pathologischer Veränderungen in einem Säuger, vermittelt durch TNF-α-umwandelnde Enzyme (TACE).

8. Verwendung gemäß Anspruch 7, wobei der behandelte Zustand Rheumatoidarthritis, Transplantatabstoßung, Kachexie, Anorexie, Entzündung, Fieber, Insulinresistenz, septischer Schock, kongestives Herzversagen, Entzündungserkrankung des zentralen Nervensystems, entzündliche Darmerkrankung oder HIV-Infektion ist.

9. Pharmazeutische Zusammensetzung, welche einen pharmazeutischen Träger und eine Matrix-Metalloproteinase oder TACE hemmende Verbindung gemäß Anspruch 1 umfasst.

10. Pharmazeutische Zusammensetzung, welche einen pharmazeutischen Träger und eine Verbindung gemäß einem der Ansprüche 1 bis 3 umfasst.

11. Verbindung, wie in einem der Ansprüche 1 bis 3 beansprucht, zur Verwendung als Arzneimittel.

12. Verfahren für die Herstellung einer Verbindung, wie in Anspruch 1 beansprucht, welche umfasst: Umsetzen einer Verbindung der Formel: 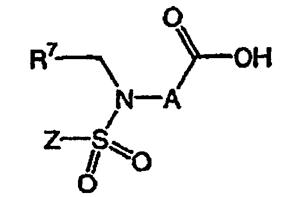 worin Z, A und R⁷ wie in Anspruch 1 definiert sind, mit Hydroxylamin.

## Revendications

1. Composé ayant la formule : 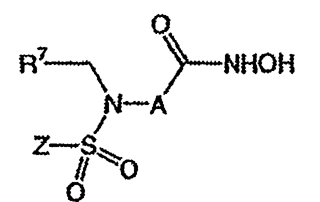 où la partie acide hydroxamique et la partie sulfonamido sont liées aux carbones adjacents du groupe A où :
A est un hétérocycle de 5 à 7 membres, monocyclique, non aromatique ayant 1 à 2 hétéroatomes choisis indépendamment parmi N, O et S, facultativement substitués par R¹, R², R³ et R⁴ ; un cycloalkyle en C₃-C₇ contenant 0-2 doubles liaisons et facultativement substitué avec R¹, R², R³ et R⁴ ; ou -CHR⁵=CHR⁶- ;
Z est aryle, hétéroaryle ou hétéroaryle fusionné avec un phényle,
où aryle est phényle ou naphtyle facultativement substitué par R¹, R², R³ et R⁴ ;
l'hétéroaryle est un hétérocycle aromatique de 5-6 membres ayant de 1 à 3 hétéroatomes choisis indépendamment parmi N, O et S et facultativement substitué par R¹, R², R³ et R⁴ ; et quand l'hétéroaryle est fusionné avec un phényle, un
ou les deux cycles peuvent être facultativement substitués par R¹, R², R³ et R⁴ ;
R¹, R², R³ et R⁴ sont indépendamment -H, -COR⁵, -F, -Cl, -I, -C(O)NR⁵OR⁶, -CN, -OR⁵, perfluoroalkyle en C₁-C₄, -S(O)ₓR⁵ où x est 0-2, -OPO(OR⁵)OR⁶, -PO(OR⁶)R⁵, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, -NR⁵C(=N⁶)NR⁵R⁶, un cyclohétéroalkyle de 3-6 membres ayant un à trois hétéroatomes indépendamment choisis parmi N, O et S, facultativement ayant 1 ou 2 doubles liaisons et facultativement substitué par un à trois groupements chacun choisi indépendamment parmi R⁵ ; -aryle ou hétéroaryle comme défini ci-dessus, -SO₂NHCOR⁵ ou -CONHSO₂R⁵ où R⁵ n'est pas H, -tétrazol-5-yle, -SO₂NHCN, -SO₂NHCONR⁵R⁶ ou alkyle en C₁-C₆, alcényle en C₂-C₆, alcynyle en C₂-C₆ à chaîne droite ou ramifiée ou cycloalkyle en C₃-C₆ ayant facultativement 1 ou 2 double liaisons chacun facultativement substitué avec -COR⁵, -CN, -alcényle en C₂-C₆, alcynyle en C₂-C₆, -OR⁵, perfluoroalkyle en C₁-C₄, -S(O)ₓR⁵ où x est 0-2, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, cycloalkyle en C₃-C₆ comme défini ci-dessus, cyclohétéroalkyle de 3-6 membres comme défini ci-dessus, aryle ou hétéroaryle comme défini ci-dessus, -SO₂NHCOR⁵ ou -CONHSO₂R⁵ où R⁵ n'est pas hydrogène, -PO(OR⁵)OR⁶, -PO(OR⁶)R⁵, -tétrazol-5-yle, -C(O)NR⁵OR⁶, -NR⁵C(=NR⁶)NR⁵R⁶, -SO₂NHCONR⁵R⁶ ou -SO₂NHCN ;
à condition que, quand R¹ et R² sont sur des carbones adjacents de A, R¹ et R² conjointement avec les carbones auxquels ils sont attachés, ils peuvent former un hétérocycle de 5-7 membres saturé ou insaturé non aromatique monocyclique, ou un hétéroaryle cyclique de 5-6 membres, chacun ayant 1 à 2 hétéroatomes indépendamment choisis parmi N, O et S, dans lequel ledit hétérocycle ou hétéroaryle cyclique peut être facultativement substitué par un à quatre groupements chacun choisi indépendamment parmi R⁴ ; ou R¹ et R² conjointement avec les carbones auxquels ils sont attachés peuvent former un carbocycle ou un cycle aryle de 5-7 membres saturé ou insaturé dans lequel ledit carbocycle ou le cycle aryle peut être facultativement substitué par un à quatre groupements chacun choisi indépendamment parmi R⁴ ;
R⁵ et R⁶ sont indépendamment définis comme H, aryle et hétéroaryle comme défini ci-dessus, cycloalkyle en C₃-C₆ comme défini ci-dessus, cyclohétéroalkyle en C₃-C₆ comme défini ci-dessus, perfluoroalkyle en C₁-C₄, ou alkyle en C₁-C₆, alcényle en C₂-C₆ ou alcynyle en C₂-C₆ à chaîne droite ou ramifiée chacun facultativement substitué avec
-OH, -COR⁸, -CN, -C(O)NR⁶OR⁹, alcényle en C₂-C₆, alcynyle en C₂-C₆, -OR⁸, perfluoroalkyle en C₁-C₄, -S(O)ₓR⁸ où x est 0-2, -OPO(OR⁸)OR⁹, -PO(OR⁸)R⁹, -OC(O)NR⁸R⁹, -COOR⁸, -CONR⁸R⁹, -SO₃H, -NR⁸R⁹, -NCOR⁸R⁹, -NR⁸COOR⁹, -SO₂NR⁸R⁹, -NO₂, -N(R⁸)SO₂R⁹, -NR⁸CONR⁸R⁹, cycloalkyle en C₃-C₆ comme défini ci-dessus, cyclohétéroalkyle en C₃-C₆ comme défini ci-dessus, aryle ou hétéroaryle comme défini ci-dessus, -SO₂NHCOR⁸ ou -CONHSO₂R⁸ où R⁸ n'est pas hydrogène, -tétrazol-5-yle, -NR⁸C(=NR⁹)NR⁸R⁹, -SO₂NHCONR⁸R⁹, -SO₂NHCN ;
R⁷ est hydrogène, alkyle en C₁-C₆, alcényle en C₂-C₆ ou alcynyle en C₂-C₆ à chaîne droite ou ramifiée, chacun facultativement substitué avec
-OH, -COR⁵, -CN, alcényle en C₂-C₆, alcynyle en C₂-C₆, -OR⁵, perfluoroalkyle en C₁-C₄, -S(O)ₓR⁵ où x est 0-2, -OPO(OR⁵)OR⁶, -PO(OR⁵)R⁶, -OC(O)NR⁵R⁶, -COOR⁵, -CONR⁵R⁶, -SO₃H, -NR⁵R⁶, -NR⁵COR⁶, -NR⁵COOR⁶, -SO₂NR⁵R⁶, -NO₂, -N(R⁵)SO₂R⁶, -NR⁵CONR⁵R⁶, cycloalkyle en C₃-C₆ comme défini ci-dessus, cyclohétéroalkyle en C₃-C₆ comme défini ci-dessus, aryle ou hétéroaryle comme défini ci-dessus, -SO₂NHCOR⁵ ou -CONHSO₂R⁵ où R⁵ n'est pas hydrogène, -tétrazol-5-yle, -NR⁵C(=NR⁶)NR⁵R⁶, -C(O)NR⁵R⁶, -SO₂NHCONR⁵R⁶, -SO₂NHCN ;
ou R⁷ est phényle ou naphtyle, facultativement substitué par R¹, R², R³ et R⁴ ou un groupement hétéroaryle de 5 à 6 membres ayant 1 à 3 hétéroatomes choisis indépendamment parmi N, O et S et facultativement substitué par R¹, R², R³ et R⁴ ;
ou R⁷ est cycloalkyle en C₃-C₆ ou cyclohétéroalkyle de 3-6 membres comme défini ci-dessus ;
ou R⁷CH₂-N-A-, où A est comme défini ci-dessus, peut former avec un atome de carbone de A adjacent au carbone portant le groupement sulfonamido, un hétérocycle non aromatique fusionné de 7-10 membres contenant facultativement un hétéroatome supplémentaire choisi parmi O, S et N dans lequel l'hétérocycle peut être facultativement fusionné à un cycle benzène ;
R⁸ et R⁹ sont indépendamment H, aryle ou hétéroaryle comme défini ci-dessus, cycloalkyle ou cyclohétéroalkyle en C₃-C₇ comme défini ci-dessus, perfluoroalkyle en C₁-C₄, alkyle en C₁-C₆, alcényle en C₂-C₆ ou alcynyle en C₂-C₆ à chaîne droite ou ramifiée, chacun facultativement substitué avec hydroxy, alkoxy, aryloxy, perfluoroalkyle en C₁-C₄, amino, mono- et di-alkylamino en C₁-C₆, acide carboxylique, carboalkoxy et carboaryloxy, nitro, cyano, carboxamido primaire, mono- et dialkylcarbamoyle en C₁-C₆ ;
un isomère optique ou un diastéréoisomère de celui-ci ;
ou un sel pharmaceutiquement acceptable de celui-ci.

2. Composé selon la revendication 1 dans lequel le groupement Z est un para-alkoxyphényle, para-aryloxyphényle ou para-hétéroaryloxyphényle.

3. Composé selon la revendication 2 qui est choisi parmi le groupe constitué de :
l'hydroxyamide de l'acide (trans)-2-[benzyl-(4-méthoxybenzènesulfonyl)amino]cyclohexanecarboxylique et
l'hydroxyamide de l'acide (cis)-2-[benzyl-(4-méthoxybenzènesulfonyl)amino]cyclohexanecarboxylique.

4. Utilisation d'un composé selon l'une quelconque des revendications 1 à 3 dans la préparation d'un médicament pour inhiber chez un mammifère les changements pathologiques occasionnés par les métalloprotéinases matricielles.

5. Utilisation selon la revendication 4 dans laquelle le trouble traité est l'athérosclérose, la formation de la plaque athéroscléreuse, la réduction de la thrombose coronaire à partir de la rupture de la plaque athéroscléreuse, la resténose, les ostéopénies occasionnées par MMP, les maladies inflammatoires du système nerveux central, le vieillissement de la peau, l'angiogenèse, la métastase tumorale, la croissance tumorale, l'ostéoarthrite, l'arthrite rhumatoïde, l'arthrite septique, l'ulcération de la cornée, le retard de cicatrisation, les maladies osseuses, la protéinurie, la maladie aortique anévrismale, la perte dégénérative du cartilage après lésion articulaire traumatique, les maladies démyélinisantes du système nerveux, la cirrhose du foie, la maladie glomérulaire du rein, la rupture prématurée des membranes foetales, les maladies inflammatoires intestinales ou la maladie périodontale.

6. Utilisation selon la revendication 4 dans laquelle le trouble traité est la dégénérescence maculaire liée à l'âge, la rétinopathie diabétique, la vitréorétinopathie proliférative, la rétinopathie de la prématurité, l'inflammation oculaire, le kératocône, le syndrome de Sjogren, la myopie, les tumeurs oculaires, l'angiogenèse oculaire/néovascularisation et le rejet de greffe de cornée.

7. Utilisation selon l'une quelconque des revendications 1 à 3 dans la préparation d'un médicament pour inhiber chez un mammifère les changements pathologiques occasionnés par l'enzyme de conversion du TNF-α (TACE).

8. Utilisation selon la revendication 7 dans laquelle le trouble traité est l'arthrite rhumatoïde, le rejet de greffe, la cachexie, l'anorexie, l'inflammation, la fièvre, la résistance à l'insuline, le choc septique, l'insuffisance cardiaque congestive, la maladie inflammatoire du système nerveux central, les maladies inflammatoires intestinales ou l'infection par le HIV.

9. Composition pharmaceutique comprenant un support pharmaceutique et une métalloprotéinase matricielle ou un composé inhibant la TACE selon la revendication 1.

10. Composition pharmaceutique comprenant un support pharmaceutique et un composé selon l'une quelconque des revendications 1 à 3.

11. Composé selon l'une quelconque des revendications 1 à 3 pour l'utilisation comme médicament.

12. Procédé pour la préparation d'un composé selon la revendication 1 qui comprend la réaction d'un composé de formule : 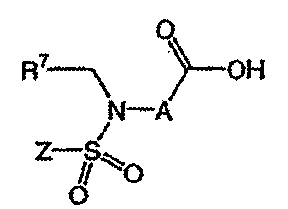 dans laquelle Z, A et R⁷ sont comme définis dans la revendication 1 avec l'hydroxylamine.
